# EUROPEAN PATENT SPECIFICATION

(11) **EP 1 984 734 B1**
(45) Date of publication and mention of the grant of the patent: **07.05.2014**
(21) Application number: 07700542.9
(22) Date of filing: 05.01.2007
(51) Int. Cl.: C07D 295/096, A61K 31/495, G01N 33/50, G01N 33/574, G01N 33/68

(54) **SIGMA-2 RECEPTOR, METHOD OF SCREENING OF SPECIFIC LIGANDS AND USE OF THE SAME IN DIAGNOSTIC OR THERAPEUTIC METHODS**
SIGMA-2-REZEPTOR, VERFAHREN ZUM SCREENING FÜR SPEZIFISCHE LIGANDEN UND ANWENDUNG DAVON BEI DIAGNOSTISCHEN ODER THERAPEUTISCHEN VERFAHREN
RECEPTEUR SIGMA 2, PROCEDE DE CRIBLAGE DE LIGANDS SPECIFIQUES ET UTILISATION DESDITS LIGANDS POUR DES PROCEDES DIAGNOSTIQUES OU THERAPEUTIQUES

(30) Priority: 05.01.2006 IT RM20060007
(43) Date of publication of application: 29.10.2008
(73) Proprietor: Universita' degli Studi di Bari, 70121 Bari (IT)
(72) Inventor: COLABUFO, Nicola Antonio, 70125 Bari BA (IT); BERARDI, Francesco, 70125 Bari BA (IT); PERRONE, Roberto, 70125 Bari BA (IT)
(74) Representative: Germinario, Claudio
(86) International application number: PCT/IB2007/050029
(87) International publication number: WO 2007/077543

(56) References cited:
- COLABUFO N. A. ET AL.: "Is the sigma2 receptor a histone binding protein?" JOURNAL OF MEDICINAL CHEMISTRY, vol. 49, no. 14, 13 July 2006 (2006-07-13), pages 4153-4158, XP009085317 United States ISSN: 0022-2623
- SUKA N. ET AL.: "Highly specific antibodies determine histone acetylation site usage in yeast heterochromatin and euchromatin." MOLECULAR CELL, vol. 8, no. 2, August 2001 (2001-08), pages 473-479, XP009085454 ISSN: 1097-2765
- BRAUNSTEIN M ET AL: "Efficient transcriptional silencing in Saccharomyces cerevisiae requires a heterochromatin histone acetylation pattern." MOLECULAR AND CELLULAR BIOLOGY, vol. 16, no. 8, August 1996 (1996-08), pages 4349-4356, XP009085458 ISSN: 0270-7306
- CLEMENTS C. ET AL.: "Crystal structure of HLA-G: a nonclassical MHC class I molecule expressed at the fetal-maternal interface." PROCEEDINGS OF THE NATIONAL ACADEMY OF SCIENCES OF THE UNITED STATES OF AMERICA, vol. 102, no. 9, 1 March 2005 (2005-03-01), pages 3360-3365, XP003007722 ISSN: 0027-8424
- BERARDI F ET AL: "4-(Tetralin-1-yl)- and 4-(Naphthalen-1-yl)alkyl Derivatives of 1-Cyclohexylpiperazine as s Receptor Ligands with Agonist s2 Activity" JOURNAL OF MEDICINAL CHEMISTRY,, vol. 47, no. 9, 30 March 2004 (2004-03-30), pages 2308-2317, XP002398146 AMERICAN CHEMICAL SOCIETY. WASHINGTON, US ISSN: 0022-2623 cited in the application
- BERARDI F. ET AL.: "N-(omega-(tetralin-1-yl)alkyl) derivatives of 3,3-dimethylpiperidine are highly potent and selective sigma1 or sigma2 ligands" JOURNAL OF MEDICINAL CHEMISTRY, vol. 41, no. 21, 8 October 1998 (1998-10-08), pages 3940-3947, XP009085320 ISSN: 0022-2623
- KIKUCHI TAKEFUMI ET AL: "INACTIVATION OF P57KIP2 BY REGIONAL PROMOTER HYPERMETHYLATION AND HISTONE DEACETYLATION IN HUMAN TUMORS", ONCOGENE, NATURE PUBLISHING GROUP, GB, vol. 21, no. 17, 18 April 2002 (2002-04-18), pages 2741-2749, XP009085448, ISSN: 0950-9232, DOI: DOI:10.1038/SJ.ONC.1205376
- HESS-STUMPP H: "Histone deacetylase inhibitors and cancer: from cell biology to the clinic", EUROPEAN JOURNAL OF CELL BIOLOGY, WISSENSCHAFLICHE VERLAGSGESELLSCHAFT, STUTTGART, DE, vol. 84, no. 2-3, 9 March 2005 (2005-03-09), pages 109-121, XP004954663, ISSN: 0171-9335, DOI: DOI:10.1016/J.EJCB.2004.12.010

## Description

### Field of the invention

The present invention relates to the elucidation of the chemical nature of the cellular sigma-2 receptor, in particular to the identification, isolation and characterization of the proteins forming the receptor. The invention further relates to the utilization of said proteins for preparing a screening assay of specific candidate ligands for the sigma-2 receptor, as well as to the use of the ligands for setting up diagnostic assays for tumour tissues and for preparing antitumour drugs. Lastly, the isolated proteins are utilized for producing anti-receptor antibodies, which likewise find employ in the diagnosis and therapeutic treatment of neoplasias.

### State of the prior art

Sigma receptors are classified as sigma-1 and sigma-2 and are localized in different tissues, including the central and peripheral nervous system. It is known in literature that expression of sigma-1 and -2 receptors in normal tissues is less than in tumour tissues. These receptors are overexpressed in several human and animal tumour cell lines, among which human neuroblastoma cell lines of SK-N-SH type.

To date, the sigma-1 receptor has been isolated and cloned, whereas the sigma-2 subtype could not be isolated and characterized yet. Moreover, though diagnostic and therapeutic applicability of the sigma-2 receptor has been suggested, the role of this receptor and the mechanism of overexpression in the tumour tissue still await elucidation.

In view of the high expression levels of the sigma-2 receptor in several tumour tissues, potent and selective sigma-2 ligands might be used as markers in diagnostic techniques such as the PET or SPECT methods.

In the last years, the present authors have developed several ligands for the sigma-2 receptor, and among them a cyclohexylpiperazine derivative PB28, whose formula is reported in diagram I. PB28 exhibits high affinity and a potent agonist activity (Perrone et al. 2000, Med Chem Res, 10, pp. 201-207; Berardi et al. 1996, J.Med Chem.39, pp. 176-182).

Although sigma-2 ligands are deemed to be potential tools for cancer diagnosis and may open up novel therapeutic perspectives, several aspects need to be clarified that are essential in order to achieve practical applications. In fact, to date the neurotransmitter of the sigma-2 receptor is still unknown, nor has been elucidated the mechanism involved in the activation of the receptor itself. Advances in this direction have been hindered by the fact that the protein(s) performing the function of sigma-2 receptor have not been isolated and characterized.

Hence, scope of the present invention is to identify the chemical nature of the sigma-2 receptor, making available novel instruments for diagnosis and therapy of neoplastic manifestations.

### Summary of the invention

The present invention is based on the elucidation of the chemical nature of the sigma-2 receptor, to date unknown, and on the identification of the proteins individually or collectively involved in the sigma-2 receptor function. These proteins are the human histones: H3, H2B, H2A.5, H1, H2.1, and the human 40S ribosomal protein S3.

Sigma-2 receptor overexpression in tumour tissues corresponds to the histone present in deacetylated form. Such a deacetylated form of the histones causes the production of new lysine NH₂-residues and the consequent binding to sigma-2 ligands. This observation has opened up new perspectives for cancer therapy based on the inhibition of histone deacetylase enzymes (Mork et al. Curr. Pharm Des. 2005, N. 11, pp. 1091-1094; Hess-Stumpp et al. Eur. J. Cell Biol. 2005, 84, pp. 109-121).

The discovery has allowed the setting up of methods of screening and identifying novel ligands specific for the sigma-2 receptor.

Accordingly, an object of a first embodiment of the invention is a method of preparing of specific ligands for a sigma-2 type receptor comprising steps of
- contacting candidate ligand compounds with one or more human histones in a partially deacetylated form selected from histones H3, H2B, H2A.5, H1, H2.1 and/or human 40S ribosomal protein S3;
- singling out the compound capable of generating a receptor/ligand complex;
- isolating the ligand from the complex, and, optionally,
- purifying and identifying the ligand.

The one or more histones and/or the human 40S ribosomal protein S3, are all in a form selected from the pure form, the cell membrane fraction, the nuclear protein fraction or intact natural or genetically modified cells.

In particular the receptor, either in the form of mixture of pure proteins or cell fraction or intact cell, is in liquid phase or is immobilized on a stationary phase.

When a candidate is individually capable of forming a complex, then the compound represents a sigma-2 ligand.

Object of a second embodiment of the invention is a family of novel ligands specific for the sigma-2 receptor and identified through the above-mentioned method of screening.

A third object of the present invention are diagnostic probes comprising complexes comprised of a ligand and a marker molecule capable of emitting a preferably fluorescent recognizable signal.

A fourth object of the invention are *in vivo* and *in vitro* diagnostic methods for the detection of the presence and the monitoring of the development of tumour tissues or other tissues, capable of overexpressing the sigma-2 receptor via the use of the probes of the invention.

Further objects are the medical applications and therapeutic compositions comprising the ligands of the invention, individually or collectively for the treatment of neoplastic forms modulated by the activation of the sigma-2 receptor.

The invention, which stems from the identification of proteins corresponding to the sigma-2 receptor, previously only known as a mere biological function, consists in the various therapeutic/diagnostic applications previously unforeseeable due to lack of knowledge of the proteins responsible for said function.

### Description of the figures

Figure 1: The figure reports the results of SDS-PAGE Gel Electrophoresis analysis of the affinity column-eluted protein.
Figure 2: The figure illustrates the saturation curves representative for [³H]-DTG on the sigma-2 receptor in the eluted membrane fraction (A) and nuclear fraction (B). The constants *K*_{d} (nM) and *B*ₘₐₓ (fmol/mg of protein) have been obtained from the specific binding curves (*P<0.0001*). The values are average values ± s.e.m. of three experiments conducted in triplicate. Data sets were compared by variance analysis according to Turky's multiple comparison test (total binding vs specific binding, total binding vs nonspecific binding, nonspecific binding vs specific binding; *P<0.0001*).
Figure 3: The figure depicts the planar structure of the ligands of formula 1.

### DETAILED DESCRIPTION OF THE INVENTION

Any time human H3, H2B, H2A.5, H1 and H2.1 histones are cited in the following text, the corresponding "partially deacetylated" histones are meant.

### Identification of proteins responsible for the sigma-2 receptor function.

It is known that SK-N-SH human neuroblastoma cells express high levels of sigma-2 receptor, and that the PB28 compound represents the most potent and selective ligand for said receptor. These properties have been utilized to develop an affinity chromatography method to isolate the protein(s) responsible for the sigma-2 receptor function.

For this purpose, the PB28 compound was functionalized by introducing an amino group on the aromatic ring (compound 2), subsequently exploited for the immobilization, through covalent bond, onto the stationary solid matrix (see diagram 1 reported below). The amino group was introduced at a position on the tetraline nucleus that is unaffected, as demonstrated in previous studies (Berardi et al. 2004, J. Med. Chem. 47, pp. 2308-2317) by insertion of substituents, so that the affinity and the sigma-2 activity of the amino-substituted ligand proved unmodified. Compound 2 was immobilized on a matrix activated with exiting N-hydroxysuccinimide groups.

Subsequently, a lysate of human neuroblastoma cells was prepared by osmotic lysis and loaded on the affinity matrix as indicated above. Then, the protein fraction was eluted with buffer.

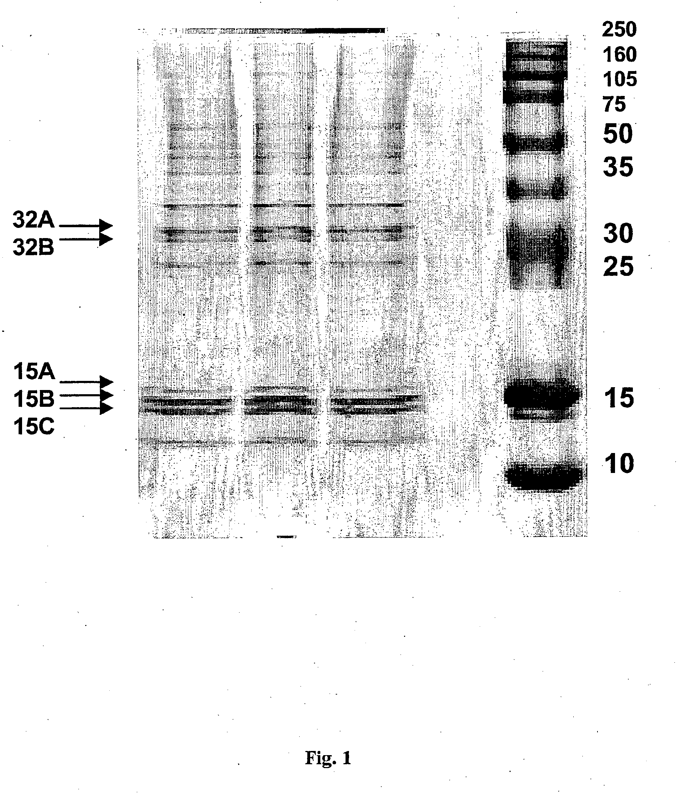

Eluate analysis by SDS-PAGE gel electrophoresis (Figure 1) highlights several protein bands, among which bands 15A, 15B and 15C of apparent molecular weight ranging from 10 kDa to 15 kDa, whereas bands 32A and 32B exhibit an apparent molecular weight ranging from 30 kDa to 35 kDa. The bands removed from the gel were hydrolyzed *in situ* with the known procedure, and each peptide mixture directly identified through MALDI-MS analysis. Measured molecular mass values were utilized for a search in the NCBI and/or SwissProt protein material database with the aid of the MASCOT program for protein identification through interpretation of peptide mass fingerprint. This strategy allowed to identify the nature of the proteins with a molecular weight of 15 kDa. Specifically, protein 15A was identified as the human histone H3 (NCBI accession number 51859316), protein 15B as the human histone H2B (NCBI accession number 1568557), protein 15C as the human histone H2A.5 (NCBI accession number 70686).

Identification of 32-kDa bands required further analyses. Samples of said bands were subjected to LC-MS-MS mass spectrometry and reverse-phase chromatography. Then, information obtained was utilized for a research on protein databases with the MASCOT MS-MS ion search program, which allowed to identify the main component of the protein band 32A as the human histone H1 (NCBI accession number (identification) 22770677), whereas band 32B exhibited/displayed a main component corresponding to human 40S ribosomal protein S3 (SwissProt accession number P23396) and an accessory component corresponding to human histone H2.1 (SwissProt accession number P16403).

The mass and the number of amino acid residues of each peptide analyzed are listed in Table 1.

**Table 1**

| Band | protein | Mass (Da) | a.a. | NCBI identification | SwissProt accession number |
|---|---|---|---|---|---|
| | | | | | |
| 15A | Histone H3.3A | 15285 | 136 | 51859376 | Q66I33 |
| 15B | Histone H2B | 13775 | 125 | 1568557 | Q93080 |
| 15C | Histone H2A.5 | 14053 | 129 | 70686 | |
| | | | | | |
| 32A | Histone H1 | 22565 | 226 | 22770677 | P16401 |
| | | | | | |
| 32B | Histone H2.1 | 21234 | 212 | | P16403 |
| | 40S ribosomal protein S3 | 26688 | 243 | | P23396 |

Results were confirmed by saturation binding analysis.

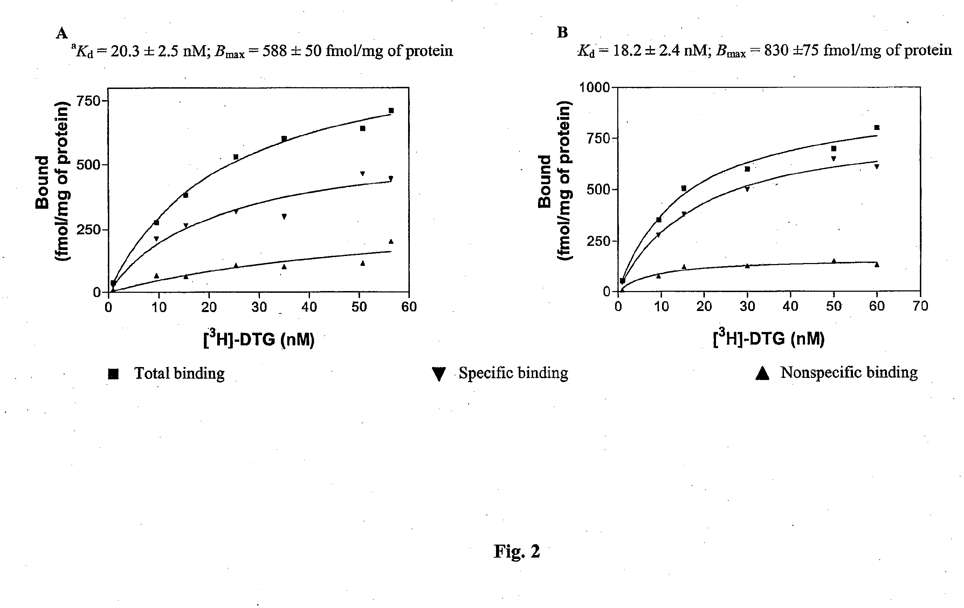

The proteins, eluted by affinity chromatography, dialyzed and lyophilized, were suspended in incubation buffer for assessment by specific saturation binding analysis with [³H]-DTG (figure 2). As reported in figure 2A, the calculated values of K_{d} equal to 20.3 ± 2.5 nM and of Bₘₐₓ equal to 588 ± 50 fmol/mg of protein were in accordance with corresponding values previously calculated on SK-N-SH neuroblastoma cell membrane preparations (K_{d} of 21.0 ± 2.0 nM and Bₘₐₓ of 656 ± 25 fmol/mg of protein). Since histones represent the nuclear protein fraction, a raw nuclear protein fraction from SK-N-SH neuroblastoma was prepared, to be subjected to the same specific saturation binding analysis with [³H]-DTG. The results are illustrated in Figure 2B and show values in accordance with the observed results, both with eluted proteins and raw cell membrane preparations.

### Sigma-2 histones

In eukariotic cells, orderly DNA packaging in the nucleus plays a significant role in the regulation of gene transcription. Nuclear DNA is ordered in a complex compact structure, chromatin, whose core is comprised of an octamer of highly conserved basic proteins: the histones.

The histones performing the function of sigma-2 receptor according to the invention, H3, H2B, H2A.5, H1, H2.1, and human 40S ribosomal protein S3, are all well-known proteins, widely described in literature; their sequences are available from public databases, as reported in Table 1. Likewise, it is known that the amino-terminal ends of the histones are subjected to post-translational modifications, in particular by acetylation/deacetylation of lysine residues by enzymes histone deacetylases (HDACs) and histone acetyltransferases (HATs). These determine the degree of acetylation of the histones and are somehow involved in the regulation of gene expression.

In non-tumour cells, sigma-2 histones are expressed at moderate levels and in an essentially acetylated form. On the contrary, it has been observed that in tumour cells there is overexpression of the same sigma-2 histones in a strongly deacetylated form; this creates a wider possibility of binding between the histone and the sigma-2 ligand(s).

Tumour tissue types exhibiting these characteristics are, e.g., human neuroblastoma, human urothelial carcinoma, human mammary carcinoma, rat glioma.

Activation of the sigma-2 receptor by known ligands blocks cell cytodieresis in the G0/G1 phase, thereby driving the same cell into apoptosis (cellular death) by a mechanism to date not clearly defined (Colabufo et al. Naunyn Schmiedeberg's Arch. Pharmacol. 2004, 370, pp. 106-113).

Moreover, overexpression of sigma-2 receptors in tumour cells makes said receptors tumour-specific markers. Therefore, novel sigma-2 agonists can find application both in the field of the diagnosis of neoplasias and in that of treatment of the same.

For this purpose, in accordance with the present invention there have been set up methods of identifying novel sigma-2 agonists based on receptor/ligand interaction assays.

### Method of screening and preparing of novel ligands

The method of preparing novel ligands according to the invention provides a) a step of screening, comprising a contacting of candidate ligands with one or more human histones selected from histones H3, H2B, H2A.5, H1, H2.1 and/or human 40S ribosomal protein S3; b) a step of singling out whether such a candidate has formed a receptor/ligand complex; c) a step of isolating the histone and, if necessary, other steps of purifying and identifying the individual ligand compound.

The production of the candidate ligand compounds, which is not part of the present invention, is conducted in accordance with known drug-design, molecular modelling or combinatorial synthesis methodologies and criteria. The compounds thus obtained can be subjected to screening in accordance with the present invention, both in the form of individual compounds and in the form of combinatorial mixtures or libraries.

The sigma-2 histones and the 40S ribosomal protein S3 utilized as receptors in the method according to the invention may be utilized both individually and in a mixture of two or more elements.

It is only thanks to the contribution of the present invention that the chemical nature of the sigma-2 has been clarified, and that it is possible to set up methods of screening utilizing as receptors pure, individually isolated proteins, as well as those cell fractions known to contain the histones and the 40S ribosomal protein, i.e. the membrane fraction or the nuclear protein fraction. However, also intact cells capable of naturally expressing high levels of histones and 40S ribosomal protein S3, or host cells genetically modified to express the same as heterologous membrane proteins may be utilized as receptors in the methods of the invention.

Each of these forms was utilized in a physical state allowing the recognizing of the formation of the receptor/ligand complex: e.g., in a solution, suspension or immobilized on a stationary phase. Whole cells were cultivated up to at least partial confluence, and utilized directly in a form adhered onto the culture medium or, alternatively, after removal from the culture medium, in the form of a suspension.

After incubation with the candidate ligands for a sufficient time span, from 60 to 150 min, formation of a histone/ligand complex was highlighted. The formation of the histone/ligand complex was monitored through kinetic studies of complex association/disassociation, with working protocols already reported in literature (Colabufo et al. 2001, Eur. J. Pharmacol. 427, pp.1-5).

The formation of a complex was highlighted through an assay in which the histone proteins were suspended in 50 mM Tris pH 8.0. Into a final volume of 1 mL buffer there were added the [³H]-DTG radioligand and various ligand concentrations. At equilibrium, determined by the above-mentioned kinetic studies, the histone/radioligand complex was separated after i20 min by filtration on glass fiber (GF/C) membranes, and quantification of the same was performed by radioactivity count. Ligand affinity for histone proteins were inversely proportional to radioactivity measured using as reference the maximum histone/[³H]-DTG radioactivity value measured in the absence of ligand.

### Family of novel ligands

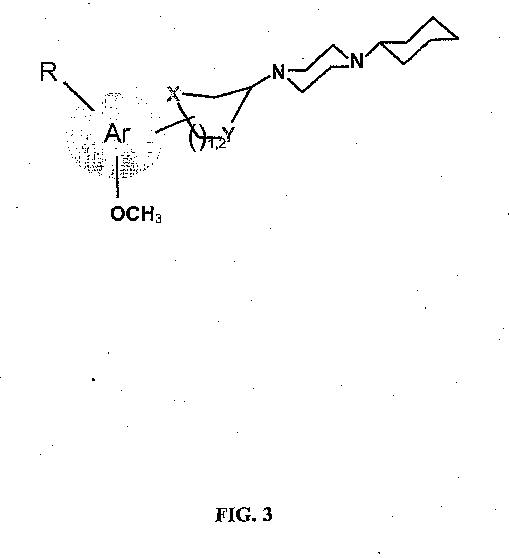

The method of screening according to the invention allowed to identify and isolate novel effective ligands belonging to a family having general formula 1 and depicted in planar form in figure 3.

The class of compounds is characterized by a 1-cyclohexylpiperazine residue substituted in the 4-position by a cyclohexyl or cyclopentyl, or a 5- or 6-member saturated heterocycle, which in turn is substituted in the 3- or 4-position with respect to the binding with piperazinic nitrogen, as in formula 1: 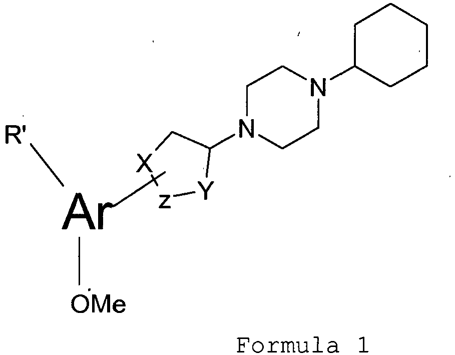
where **Ar** is a 5- or 6-member monocyclic methoxyaryl or methoxyheteroaryl group containing one or more heteroatoms selected from N, O, S, or condensated polycyclic methoxyaryl, optionally partially hydrogenated,
R' is selected from H, OH, linear or branched C1-C5 alkoxyl, linear or branched C1-C5 alkyl, linear or branched C1-C5 halogenoalkyl, halogen, NO₂, CH₂OH, NHCH₃, N(CH₃)₂, CONH₂, NHSO₂CH or COCH₃;
**Z** is -CH₂ -, -CH<, -C₂H₄ -, or -C2H3<;
X and Y may be heteroatoms N, O and S.

There are included the geometric *cis* and *trans* forms of each compound indicated.

In a specific embodiment of the invention **Ar** is a 2-methoxyphenyl, 2-methoxy-6-aminophenyl, 2-methoxy-5-aminophenyl, 2-methoxy-5-aminophenyl or 2-methoxy-6-nitrophenyl residue.There are included the geometric *cis* and *trans* forms of each compound indicated.

Examples of ligands identified with the method of the invention are:
*trans*-1-cyclohexyl-4-[4-(2-methoxy-phenyl)cyclohexyl]piperazine;
*trans*-1-cyclohexyl-4-[4-(2-methoxy-6-ethylphenyl)cyclohexyl] piperazine;
*trans*-1-cyclohexyl-4-[4-(2-methoxy-6-amino-phenyl)cyclohexyl]piperazine;
*trans*-1-cyclohexyl-4-[4-(2-methoxy-5-methylamino-phenyl)cyclohexyl]piperazine;
*trans*-1-cyclohexyl-3-[4-(2-methoxy-phenyl)cyclopentyl]piperazine;
*trans*-1-cyclohexyl-4-[5-(2-methoxy-phenyl)6-piperidyl]piperazine;
*trans*-1-cyclohexyl-4-[4-(5-methoxy-naphtyl)cyclohexyl]piperazine, or corresponding *cis* isomers.

### Biological assessment of ligands of formula 1.

The compounds indicated were subjected to competition binding assays using as biological preparation the mixture of the affinity column-eluted histone proteins (20 mcg) and, as radioligand, the [³H]-DTG compound. The affinity value (*K*ᵢ) for the *trans*-1-cyclohexyl-4-[4-(2-methoxy-phenyl)cyclohexyl]piperazine compound is of 0.068 nM.

In this biological assay, the analogous compounds belonging to the same class with *trans* isomery exhibit affinity values ranging from 0.010 nM to 10.0 nM. The corresponding *cis* isomers are found to be less affine, with *K*ᵢ values ranging from 10.0 nM to 500 nM.

### Diagnostic applications

The ligand compounds identified in accordance with the present invention possess antiproliferative and cytotoxic activity on neuroblastoma or glioma tumour cells, measured in antiproliferation and cytotoxicity assays previously described by Colabufo et al. (Naunyn-Schmiedeberg's Arch Pharmacol. 2004 370, pp. 106-1139). These results demonstrate the applicability of the compounds of the invention as antitumour agents effective on tumours characterized by high expression levels of the histone sigma-2 receptors.

Moreover, sigma-2 ligand compounds exhibiting high affinity for tumour cells, either identified in accordance with the present invention or previously known, find application as selective vectors of antitumour medicaments, as well as effective diagnostic agents for the *in vivo* or *in vitro* detection of the presence of solid tumours and the monitoring of their development.

In order to be useful as diagnostic agents, the high-affinity ligand compounds are chemically modified and bound to marker molecules capable of emitting a recognizable signal. Suitable markers are radioactive isotopes, enzymes, fluorescent compounds and any other substance well-known in the field of diagnostic assays.

As to the use of radioactive isotopes utilizable in accordance with the present invention, one of the modified PET tracers, used in tumour diagnoses, is ¹⁸F-fluorodeoxyglucose, or [¹⁸F]-FDG, which yields important information on glucide metabolism in the neoplastic tissue. In this field, other tracers allowing a more detailed mapping of the tumour region are being developed.

As to fluorescent tracers, these are widely used in flow cytofluorimetry, above all in liquid tumours (myelomas) for the characterization of tumour forms, appreciating the density/granularity, nucleus-cytoplasm ratio, cell size and optical homogeneity in the cell population.

Since the receptor-binding site is comprised in the cyclohexylpiperazine portion, the ligand compounds are functionalized on the molecule portion farthest to the active site, i.e. on the aryl radical. Several strategies are available for this purpose.

There may be utilized an amino residue already present on the aromatic portion, as in the case of the compound 1-cyclohexyl-4-[4-(2-methoxy-6-amino-phenyl)cyclohexyl]piperazine, or be introduced a nitro group that is subsequently reduced to amino group as described in diagram 1 reported below, 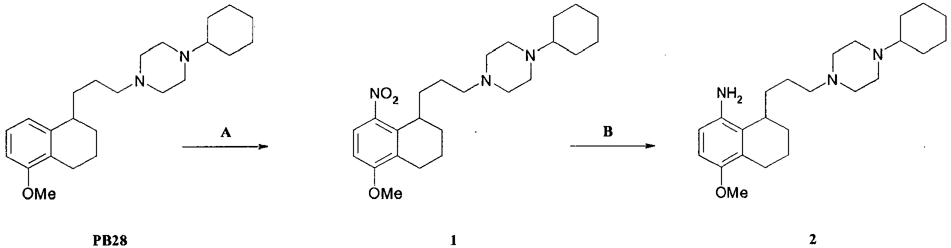
where (**A**) NO₂BF₄; (**B**) SnCl₂, HCl

Alternatively, the methoxy residue present in all compounds of formula 1 may be transformed into a hydroxyl group according to diagram 2 reported below:

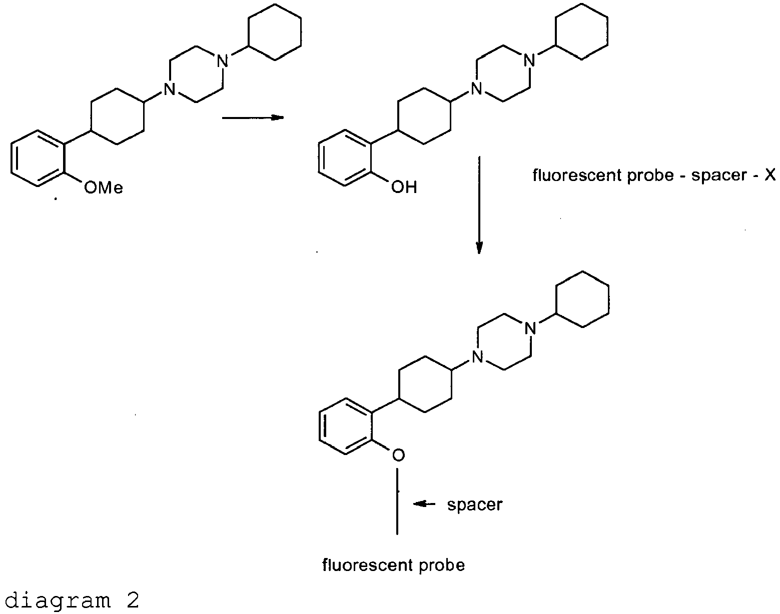

Then, the functional group thus obtained, be it NH₂ or OH or other, is utilized in the forming of the binding with the marker molecule, e.g. with a fluorescent molecule, like coumarin, preferably through a spacer.

### Therapeutic applications

The ligands of the invention find application for use as medicaments. In particular, in the treatment of neoplasias characterized by overexpression of the histones H3, H2B, H2A.5, H1, H2.1 and of the human 40S ribosomal protein S3.

Sigma-2 ligand compounds find therapeutical application in tumour treatment and development control, by virtue of their intrinsic apoptotic and cytotoxic activity on tumour cells demonstrated on glioma or neuroblastoma cells (Colabufo et al. 2004) as well as valid vectors capable of carrying to the tumour tissue antitumour medicaments or substances capable of interfering with the cellular mechanisms causing the development of the same. The sigma-2 ligands, along with other classes such as the calcium-antagonists, by having proved to be good P-Glycoprotein modulators (Kawamura et al. 2003, Synapse, 48, pp. 80-86) responsible for MDR (Multi Drug Resistance) may be utilized to carry the chemioterapic agent inside tumour cells that have become resistant following chemotherapy (Seelig et al. 2005, Mini-Reviews in Med. Chem. 5, pp 135-151). To date, the best molecules such as Tariquidar (Roe et al. 1999, 9, pp 885-892; Mistry et al. 2001, 61, pp 749-758) are in phase III.

Pharmaceutical compositions suitable for the invention comprise one or more of the ligands of the invention and a pharmaceutically acceptable excipient and, optionally, usual additives. In one embodiment of the invention, the compositions are formulated in a form suitable for the treatment of tumour cells through blocking of the cycle in the G0/G1 phase and subsequent apoptosis.

The invention also relates diagnostic probes comprising the complex of the above described ligands and a marker molecule capable of emitting a recognizable signal. The marker may be a fluorescent molecule, an enzyme, a radioactive isotope.

The probes may be used in diagnostic methods for the detection of the presence of solid tumours or for the monitoring of their development. The method may also comprise a step of determining *in vitro* on tissue biopsy the expression level of one or more human histones in a partially deacetylated form selected from histones H3, H2B, H2A.5, H1, H2.1 or the human 40S ribosomal protein S3.

In a specific embodiment of the invention the determining of the expression levels is performed by using the same above described probes.

A further application of the present invention is the production of poly- and monoclonal antibodies having specificity for one or more of the proteins of histones H3, H2B, H2A.5, H1, H2.1 and the human 40S ribosomal protein S3, or fragments thereof containing antigen sites of the same. Polyclonal and monoclonal antibodies are produced in accordance with protocols well-known to a person skilled in the art. Corresponding polyclonal antibodies produced against the whole protein of the sigma-1 receptor are commercially available and utilized for recognizing the human and animal sigma-1 receptor in immunoistochemical, western blotting and immunoprecipitation assays. The same applications of anti-histone and/or anti-40SA ribosomal protein antibodies are now made available by the present invention.

The experimental examples reported hereinafter illustrate with all necessary operative details the various steps required for carrying out the various aspects of the invention, without however limiting the scope of the protection thereof.

### Example 1:

### Identification of proteins corresponding to the sigma-2 receptor. Preparation of affinity chromatography matrix.

The synthesis of the sigma-2 ligand compound PB28, reported in diagram 1 and utilized in the preparing of the affinity matrix was previously described by Perrone et al. 2000, Med. Chem. Res. 10, pp. 201-207; Berardi et al. 1996, J.Med. Chem.39, pp. 176-182.

Nitronium tetrafluoroborate NO₂BF₄ (5.76 mmol, 0.76 g) was added to a solution of PB28 (4.8 mmol, 1.77 g) in CH₃CN (15 mL). The mixture was maintained under stirring at room temperature for 8 h. The solvent was evaporated and the reaction mixture was recollected with CH₂Cl₂ and washed with ice and water. The organic phase was dried over Na₂SO₄ and the solvent evaporated to give a mixture consisting of 8-nitro derivative, 6-nitro derivative and 6,8 di-nitro derivative. Mixture purification was performed with chromatography on flash-type column, using AcOEt, MeOH and NH₄OH (9/1/0.1%) as eluent, to afford the desired compound(1-cyclohexyl-4-[3-(5-methoxy-8-nitro-1,2,3,4-tetrahydronaphtalen-1-yl)propyl]piperazine (1) as a yellow semi-solid with a 20% yield.

¹H NMR (CDCl₃, 300 MHz) δ: 1.00-1.38 [m, 5H, cyclohexylic (C*H*H)₅]_{,} 1.40-2.00 [m, 13H, (C*H₂*)₂CH (C*H₂*)₂, cyclohexylic (CH*H*)₅], 2.40-2.50 (m, 3H, CHN and CH₂N), 2.62-2.98 (m, 11H, piperazinic, benzylic CH and CH₂), 3.92 (s, 3H, OCH₃), 6.68 (d, 1H, *J* = 9.0, aromatic), 7.79 (d, 1H, *J* = 9.0, aromatic); GC-MS *m*/*z* 415 (M⁺, 3), 398 (34), 202 (100), 181 (55). ¹H-NMR signal attribution was confirmed by ¹H-NMRNOEOSY.

The compound 1-cyclohexyl-4-[3-(5-methoxy-8-nitro-1,2,3,4-tetrahydronaphtalen-1-yl)propyl]piperazine (1) (0.81 mmol, 0.34 g) dissolved in 95% EtOH (10 mL) was added dropwise to a solution containing SnCl₂ (3.24 mmol, 0.61 g) in concentrated HCl (1.8 mL). The mixture was heated to the boil for 3 h. After having cooled it, NaOH (5 N) was added and the yielded alkaline solution was extracted with Et₂O (3 x 15mL). The organic phase was washed with water and dried over Na₂SO₄. The solvent was evaporated, yielding a dark oil, purified on a flash-type chromatography column using AcOEt, MeOH and NH₄OH (9/2/0.1%) as eluent, to afford the desired compound 1-cyclohexyl-4-[3-(8-amino-5-methoxy-1,2,3,4-tetrahydronaphtalen-1-yl)propyl]piperazine (2) as a pale yellow oil, with a 50% yield.

¹H NMR (CDCl₃, 300 MHz) δ: 1.20-1.40 [m, 6H, cyclohexylic (CHN₂)₃], 1.4-0-2.00 [m, 12H, (C*H₂*)₂CH (C*H₂*)₂, cycloxylic (CH₂)₂], 2.20-2.40 (m, 3H, CHN and CH₂N), 2.54-2.76 (m, 11H, piperazinic, benzylic CH and CH₂), 3.63-3.76 (s+m, 5H, OCH₃ and NH₂ D₂O exchanged), 6.57 (d, 1H, *J* = 8.2, aromatic), 6.75 (d, 1H, *J* = 8.2, aromatic); GC-MS *m*/*z* 387 (M⁺ + 2, 3), 386 (M⁺ + 1, 17), 385 (M⁺, 61), 181 (100); ESI-MS *m*/*z* 386.3 (MH⁺). The corresponding hydrochloride salt is a white crystalline solid: m.p. = 269-274 °C; Anal. (C₂₄H₃₉N₃O·HCl·0.5 H₂O) C, H, N.

Purification of synthesis intermediates 1 and 2 was performed by flash column chromatography using 1:40 ICN silica gel 60Å (15-40 µm) as stationary phase. Melting points were determined in open capillaries with a Gallenkamp instrument. Elemental analyses (C,N,H) were performed on Eurovector Euro EA 3000 analyzer; the analytical results were within ± 0.4% of the theoretical values for the formula given. ¹H NMR spectra were recorded at 300 MHz on a Mercury Varian spectrometer, using CDCl₃ as solvent. all values are reported in ppm (δ ). Mass spectra were recorded on Agilent 6890-5973 MSD gas chromatograph/mass spectrometer and on Agilent 1100 series LC-MSD trap system VL: only significant *m*/*z* peaks, with their percentage in parentheses are reported. All spectra were in accordance with the assigned structures.

### Procedure for stationary-phase functiorialization.

The HiTrap NHS-activated stationary phase is comprised of an *N*-hydroxy-succinimmide (NHS) ester attached by an epichlorohydrine via a six-C atom spacer. This type of esterification entails an activated ester, which reacts rapidly and quantitatively with the amino group of the ligand to give a stable amide function. The activated ester is stable in the absence of water. The HiTrap NHS-activated stationary phase was provided in 100% isopropanol. For activation, the NHS-activated HiTrap was washed with cold 1 mM HCl (3 x 10 mL) by using a syringe injecting with a flow rate equal to 1 mL/min. Compound 2 (50 µmol) was solubilized in the standard buffer containing 2 M NaHCO₃, 0.5 M NaCl, at pH 8.3.

The column was sealed and maintained under these conditions for 30 min at 25 °C. The non-functionalized active sites of the column and the compound 2 not attached to the column were deactivated and discarded from the column by using Buffer **A** (0.5 M ethanolamine, 0.5 M NaCl at pH 8.3) and Buffer **B** (0.1 M acetate, 0.5 M NaCl at pH 4.0), respectively. Initially Buffer **A** (3 x 10 mL) was injected, then Buffer **B** (3 x 10 mL) then Buffer **A** (3 x 10 mL) and finally Buffer **B** (3 x 10 mL). The column was stored for subsequent use in 0.5 M Na₂HPO₄, 0.1 % NaN₃ at pH 7.0.

### Cell culture

Human neuroblastoma cells SK-N-SH were brought into culture in a medium comprised of RPMI 1640, 10% FSC, 100 U/mL penicillin, 100 µg/mL streptomycin, 1% nonessential amino acids, 1 mM sodium pyruvate and 2 mM L-glutamine under a 5% CO₂ atmosphere at 37 °C.

### Membrane preparation

The preparation of the membranes from cell line SK-N-SH was performed as described in literature (Vilner et al. 1995, Cancer Res. 55, pp. 408-413). SK-N-SH cells were brought to 80% confluence. The culture medium was removed and the cells washed with PBS. After having detached them from the plate, cells were suspended in cold 10 mM Tris-HCl buffer at pH 7.4, containing 0.32 M sucrose and treated with a Potter-Elvehjem homogenizer. The homogenate was centrifuged at 31,000 x g for 15 min at 4 °C, eliminating the supernatant. The final pellet was resuspended in 10 mM Tris-HCl buffer at pH 7.4 and frozen at - 80 °C. Protein dosage was performed by Lowry's method.

### Nuclear fraction

The nuclear fraction of SK-N-SH cells was prepared according to the method already reported in literature (Cagnotto et al. 1994, Eur. J. Pharm. 266, pp. 131-138).

SK-N-SH membranes were resuspended in a cold 10 mM Tris-HCl buffer (pH 7.4) and centrifuged at 1000 g for 10 min at 4 °C, obtaining the nuclear fraction (NP1). Final pellet (NP1) and supernatant were resuspended in buffer and stored at - 80 °C.

### Saturation analysis with radioligand

Saturation experiments were performed with procedures reported in literature (Vilner et al. *supra*). Sigma-2 receptors in the SK-N-SH membranes, in the nuclear fraction (NP1) and in the supernatant were bound by the [³H]-DTG radioligand at concentrations ranging from 1.0 nM to 150 nM. The samples, containing 400 µg of protein, radioligand, 10 µM of unlabeled DTG to determine nonspecific binding, and 1 µM of (+)-pentazocine to mask the sigma-1 receptors, were brought to equilibrium Into a final volume of 500 µL 50 mM Tris-HCl at pH 8.0 for 120 min at 25 °C. At equilibrium, 5 mL buffer (50 mM Tris-HCl, pH 7.4) were added. Samples were filtered on GF/C membranes, acclimatized in 0.5 % PEI for 30 min prior to use. Filters were washed twice with the buffer (5 mL). Radioactivity count was performed on a LS-6500 Beckman Scintillation Counter.

### Protein separation by affinity chromatography.

A suspension of SK-N-SH membranes comprised of 4 mg in 5 mL of 50 mM Tris, pH 8.0, was injected in a column having the stationary phase functionalized with the compound **2** as selector. The column was sealed and maintained at room temperature for 1 h. Subsequently, it was washed with 50 mM Tris, pH 8.0 (3 x 5 mL) to remove the biologic fraction not bound to the selector. The selector-bound biological fraction was eluted with 20 mM glycine, pH 3.0 (3 x 5 mL). The solution was lyophilized at - 52 °C and 0.061 mbar and then analyzed under SDS-PAGE gel electrophoresis. There were observed three separate bands having a mass ranging from 13 kDa to 14 kDa and a single band having a mass ranging from 29 kDa to 32 kDa.

### In situ digestion of electrophoretic bands.

Proteins present in the selector-bound biological fraction were separate by SDS-PAGE on 12% bis-acrylamide gel (Figure 1). Analysis showed several protein bands highlighted with Colloidal Comassie (Pierce) at about 15 kDa and 32 kDa. In particular, there were recognized three major bands around 15 kDa (15A, 15B, and 15C) and two bands at 32 kDa (32A and 32B). The bands were cut out, fragmented and treated with a 50 mM ammonium bicarbonate solution at pH 8.0 containing 50% acetonitrile. Gel fragments were resuspended in ammonium bicarbonate at pH 8.0, treated with DTT at 56 °C for 45 min and alkylated with a 55 mM iodoacetamide solution in the same buffer for 30 min at room temperature in a dark room. Excess reagent was eliminated; gel fragments were washed several times, then resuspended in a 50 mM ammonium carbonate buffer, incubated in 100 ng trypsin for 2 h at 4 °C and overnight at 37 °C. The peptide-containing supernatant was removed and the gel fragments washed with acetonitrile to extract the fraction of peptides still present on the gel. The two fractions were reunited and lyophilized.

### MALDI-MS Analysis

MALDI mass spectra were performed by Applied Biosystem Voyager DE-PRO. 1 µL peptide was mixed with an equal volume of α-cyano-4-hydroxycynnamic acid as matrix (10 mg/ml in 0.2% TFA in 70% acetonitrile) applied to the sample over metal plate and dry air. Mass calibration was carried out by using standard mixtures. Unprocessed data, reported as monoisotopic masses, were then input to the MASCOT search program for protein identification from protein databases (NCBI and SwissProt).

### LC-MS-MS Analysis

When peptide identity could not be determined by mere MALDI analysis, peptide mixture was analyzed by LC-MS-MS, using a Q-ToF spectrometer (Micromass, Waters). After loading, the peptide mixture (10 µL) was concentrated and washed with water, with 10 µL/min from the reverse-phase pre-column (Waters) using 0.2% formic acid as eluent. Then, the sample was separated on a C18 reverse-phase capillary column (75 µm x 20 mm) with a flow rate of 280 nL/min using a linear gradient of eluent **B** (0.2% formic acid in 95% acetonitrile) in **A** (0.2% formic acid in 5% acetonitrile) from 7% to 50% in 50 min. The mass spectrometer was set in a MS/MS mode, where a complete scanning of the spectrum (m/z acquisition from 400 to 1600 Da/e) was performed by a coupled mass spectrum (m/z acquisition from 100 to 2000 Da/e). Precursor ions were selected as three most intense peaks of the pre-scanning. Useful collision energy was applied as a function of the precursor ion mass and charge. ProteinLynx software was utilized to analyze MS and MS/MS spectra and generate a list of peaks, which was inputted to the MASCOT MS/MS search program for peptide identification.

Results allowed to identify the five separate bands in SDS-PAGE, like the histones and the 40S ribosomal protein S3 as reported in Table I.

### Example 2:

### Method of screening novel ligands

Into a final volume of 500 µL buffer (50 mM Tris, pH 8.0) there were suspended 20 µg histone proteins, the radioligand [³H]-DTG at a concentration ranging from 4.0 nM to 6.0 nM and the candidate ligand to be evaluated (screened). The sample was incubated for 120 min at 25 °C, then the histone/radioligand complex was rapidly filtered on 0 25 mm GF/C membranes. The filter was washed twice with 3 mL buffer. Scintillation liquid was added and a radioactivity reading was performed. The displacement rate was calculated as difference (%) with respect to the specific binding measures by using 10 µM of DTG.

### Evaluation of histone/radioligand complex formation: association kinetics

Into a final volume of 500 µL there were added 10 µg of mixture of eluted histone proteins and 3 nM of [³H]-DTG. Likewise, it was prepared a sample with the same amounts of protein, of radioligand and of the ligand under screening. At different times (from 5 min to 180 min) the samples were filtered in the absence and in the presence of ligand; complex formation, rate up to the reaching of the stationary state, was evaluated from the difference in radioactivity between each sample pair.

### Dissociation kinetics

Into a final volume of 500 µL there were added 10 µg eluted histone protein mixture and 3 nM [³H]-DTG. At equilibrium (120 min) the sample under screening was added, and residual radioactivity was evaluated at different times (from 5 min to 180 min). The difference in radioactivity between the stationary state and the sample filtration allowed to evaluate the histone/ligand complex dissociation event (Colabufo et al. 2001, Eur. J. Pharmacol. 427, pp.1-5)

### Example 3:

### Antiproliferative and cytotoxicity assays of the compound trans-1-cyclohexyl-4-[4-(2-methoxy-phenyl)cyclohexyl] piperazine.

The antiproliferative assay for compounds was performed by using the MTT assay (Mossman et al. 1983, J. Immunol. Methods 65, pp 55-63). The cytotoxicity assay was performed by measuring the LDH level as reported by Vilner and Bowen 2000, J. Pharmacol. Exp. Ther. 292, pp 408-413. The detailed experimental part is reported in Colabufo et al. 2004, Naunyn Schmiedeberg's Arch. Pharmacol. 370, pp. 106-113. The results observed in the two tests were homogeneous to results reported in literature for the best ligand known to date: the compound PB28.

### Example 4: Fluorescent probe

### Preparation of a fluorescent probe from the ligand trans-1-eyclohexyl-4-[4-(2-methoxy-phenyl)cyclohexyl] piperazine and the tracer 7 amino-4-methyl-coumarin.

The tracer 7-amino-4-methyl-coumarin was treated with a stoichiometric excess of a spacer of general formula X(CH₂)ₙX (where X= Cl and/or Br, n = 4-6) under reflux in acetonitrile overnight in the presence of Na₂CO₃. The solvent was evaporated and the residue recollected with 20 mL water and CHCl₃. The organic phase was dried over anhydrous Na₂SO₄. The solvent was evaporated, yielding an oil that was purified on chromatography column (CHCl₃/MeOH 95:5).

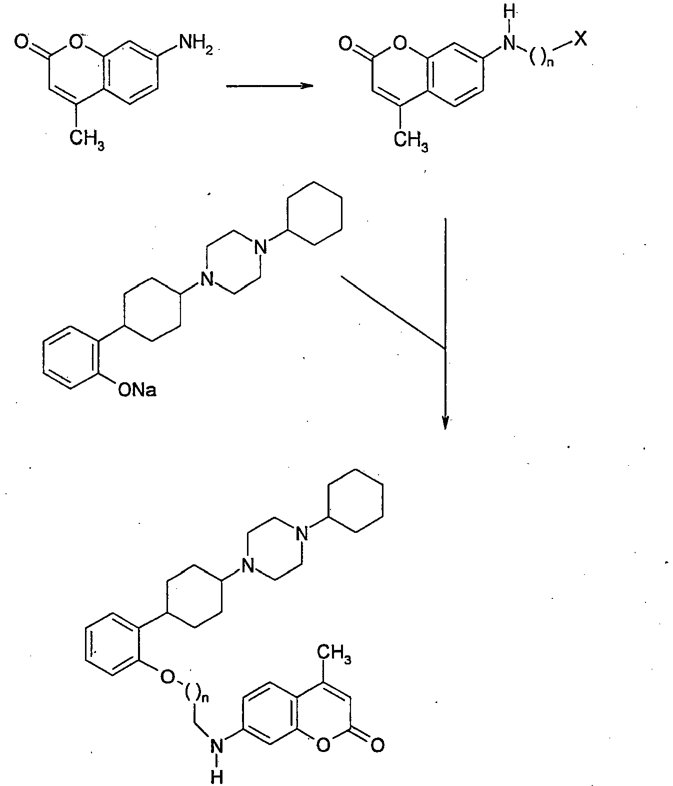

The yielded intermediate was reacted with the sodium phenate of the ligand in toluene under reflux overnight according to the above-reported scheme. The solvent was evaporated, the mixture was recollected with water and CHCl₃. The organic phase was dried over Na₂SO₄ and the solvent was evaporated. The obtained fluorescent probe was purified on a chromatography column (CHCl₃/MeOH 95:5). Spectrofluorometric working conditions: λₑₓ = 345-355, λₑₘ = 440-450.

### Example 5:

### Tumour cell Fluorescence assay:

Cells were seeded in complete medium for 48 h on a slide. The slide was treated with EtOH and subsequently washed with PBS. The slide was dipped for 2 hours in a solution containing the fluorescent probe diluted in 50 mM Tris. With the same buffer the slide was washed, and a reading was performed with λₑₓ = 345 nm and emission λₑₘ =440 nm.

## Claims

1. A method of preparing of specific ligands for a sigma-2 type receptor comprising steps of
- contacting candidate ligand compounds with one or more human histones in a partially deacetylated form selected from histones H3, H2B, H2A.5, H1, H2.1 and/or human 40S ribosomal protein S3;
- singling out the compound capable of generating a receptor/ligand complex;
- isolating the ligand from the complex, and, optionally,
- purifying and identifying the ligand
- checking the intrinsic apoptotic and cytotoxic activity of the ligand on glioma or neuroblastoma cells.

2. The method according to claim 1, wherein as receptor there are utilized one or more histones and/or the human 40S ribosomal protein S3, all in a form selected from: the pure form, the cell membrane fraction, the nuclear protein fraction or intact natural or genetically modified cells.

3. The method according to claim 2, wherein the receptor in the form of mixture of pure proteins or cell fraction or intact cell is in the liquid phase or is immobilized on a stationary phase.

4. A specific ligand for the sigma-2 receptor, obtainable through the method according to any one of the claims 1 to 4 and having general formula 1 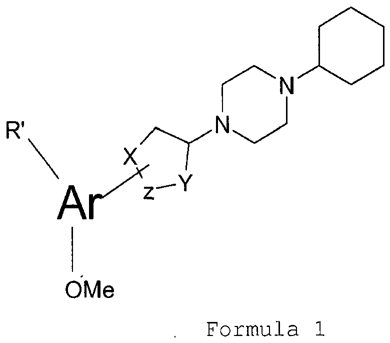 wherein:
Ar is a 5- or 6-member monocyclic methoxyaryl or methoxyheteroaryl group containing one or more heteroatoms selected from N, O, S, or condensated polycyclic methoxyaryl, optionally partially hydrogenated,
R' is selected from H, OH, linear or branched C1-C5 alkoxyl, linear or branched C1-C5 alkyl, linear or branched C1-C5 halogenoalkyl, halogen, NO₂, CH₂OH, NHCH₃, N(CH₃)₂, CONH₂, NHSO₂CH₃ or COCH₃;
**Z** is selected from -CH₂ -, -CH<, -C₂H₄ -, or -C2H3<;
**X** and **Y are** selected from N, O and S, and wherein
each cyclic unit may be in a *cis* form as well as in a *trans* form.

5. The ligand according to claim 4, wherein Ar is a 2-methoxyphenyl, 2-methoxy-6-aminophenyl, 2-methoxy-5-aminophenyl, 2-methoxy-5-aminophenyl, 2-methoxy-6-nitrophenyl residue.

6. The ligand according to claim 4, selected from:
*trans*-1-cyclohexyl-4-[4-(2-methoxy-phenyl)cyclohexyl]piperazine;
*trans*-1-cyclohexyl-4-[4-(2-methoxy-6-ethyl-phenyl)cyclohexyl]piperazine;
*trans*-1-cyclohexyl-4-[4-(2-methoxy-6-aminophenyl)cyclohexyl]piperazine;
*trans*-1-cyclohexyl-4-[4-(2-methoxy-5-methylamino-phenyl)cyclohexyl]piperazine;
*trans*-1-cyclohexyl-3-[4-(2-methoxy-phenyl)cyclopentyl]piperazine;
*trans*-1-cyclohexyl-4-[5-(2-methoxy-phenyl)6-piperidyl]piperazine;
*trans*-1-cyclohexyl-4-[4-(5-methoxy-naphtyl)cyclohexyl]piperazine, or corresponding cis isomers.

7. The ligand according to any one of the claims 4 to 6 for use as medicament.

8. The ligand according to claim 7 for use in the treatment of neoplasias **characterized by** overexpression of the histones H3, H2B, H2A.5, H1, H2.1 and of the human 40S ribosomal protein S3.

9. A pharmaceutical composition comprising one or more ligands according to claims 4 to 6, a pharmaceutically acceptable excipient and, optionally, usual additives.

10. The pharmaceutical composition according to claim 9, in a formulation suitable for the treatment of tumour cells through blocking of the cycle in the G0/G1 phase and subsequent apoptosis.

11. A diagnostic probe comprising the complex of the ligand according to any one of the claims 4 to 6 and a marker molecule capable of emitting a recognizable signal.

12. The diagnostic probe according to claim 11, wherein the marker is a fluorescent molecule, an enzyme, a radioactive isotope.

13. A diagnostic method for the detection of the presence of solid tumours or for the monitoring of their development, comprising a step of determining *in vitro* on tissue biopsy the expression level of one or more human histones in a partially deacetylated form selected from histones H3, H2B, H2A.5, H1, H2.1 and of the human 40S ribosomal protein S3, wherein the determining of the expression levels is performed by using the probe according to claim 12.

14. The method according to claim 13, wherein the tissue sample is immobilized on a solid support and the probe is a fluorescent probe.

15. The method according to any one of the claims 13 to 14, wherein the tumour is neuroblastoma, urothelial carcinoma, mammary carcinoma, glioma.

## Patentansprüche

1. Verfahren zur Herstellung von spezifischen Liganden für einen Sigma-2-Typ Rezeptor, umfassend die Schritte
- In-Kontakt-bringen von Kandidaten-Liganden-Verbindungen mit einem oder mehreren menschlichen Histonen, in einer teilweise deacetylierten Form, ausgewählt aus den Histonen H3, H2B, H2A.5, H1, H2.1 und/oder menschlichem 40S ribosomalem Protein S3;
- Auswahl der Verbindung, die einen Rezeptor/Liganden-Komplex bilden kann;
- Isolierung des Liganden aus dem Komplex und gegebenenfalls
- Aufreinigung und Identifizierung des Liganden,
- Kontrolle der intrinsischen apoptotischen und zytotoxischen Aktivität des Liganden gegenüber Gliom- oder Neuroblastom-Zellen.

2. Verfahren nach Anspruch 1, wobei als Rezeptor ein oder mehrere Histone und/oder das menschliche 40S ribosomale Protein S3 verwendet werden; alle in einer Form ausgewählt aus: der aufgereinigten Form, der Zellmembranfraktion, der Kernproteinfraktion oder intakten natürlichen oder genetisch modifizierten Zellen.

3. Verfahren nach Anspruch 2, wobei der Rezeptor in der Form eines Gemisches von aufgereinigten Proteinen oder Zellfraktion oder intakter Zelle in der Flüssigphase ist oder auf einer stationären Phase immobilisiert ist.

4. Spezifischer Ligand für den Sigma-2-Rezeptor, der erhältlich durch ein Verfahren nach einem der Ansprüche 1 bis 4 mit der allgemeinen Formel 1 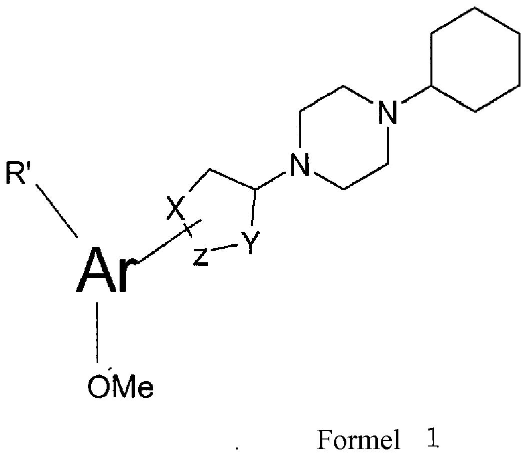 wobei:
Ar ein 5- oder 6-gliedriger monozyklischer Methoxyaryl- oder Methoxyheteroaryl-Rest, enthaltend ein oder mehrere aus N, O, S ausgewählte Heteroatome, oder ein kondensierter polyzyklischer Methoxyaryl-Rest ist, der gegebenenfalls teilweise hydriert ist,
R' aus H, OH, linearem oder verzweigtem C1-C5 Alkoxyl, linearem oder verzweigtem C1-C5 Alkyl, linearem oder verzweigtem C1-C5 Halogenalkyl, Halogen, NO₂, CH₂OH, NHCH₃, N(CH₃)₂, CONH₂, NHSO₂CH₃ oder COCH₃ ausgewählt ist;
Z aus -CH₂ -, -CH<, -C₂H₄-, oder -C₂H₃< ausgewählt ist;
X und Y aus N, 0 und S ausgewählt sind und wobei
jede zyklische Einheit in einer cis-Form sowie in einer trans-Form vorliegen kann.

5. Ligand nach Anspruch 4, wobei Ar ein 2-Methoxyphenyl-, 2-Methoxy-6-aminophenyl-, 2-Methoxy-5-aminophenyl-, 2-Methoxy-5-aminophenyl- oder 2-Methoxy-6-nitrophenyl-Rest ist.

6. Ligand nach Anspruch 4, ausgewählt aus:
*trans*-1-Cyclohexyl-4-[4-(2-methoxy-phenyl)cyclohexyl] piperazin;
*trans*-1-Cyclohexyl-4-[4-(2-methoxy-6-ethyl-phenyl)cyclohexyl] piperazin;
*trans*-1-Cyclohexyl-4-[4-(2-methoxy-6-amino-phenyl)cyclohexyl] piperazin;
*trans*-1-Cyclohexyl-4-[4-(2-methoxy-5-methylamino-phenyl)cyclohexyl] piperazin;
*trans*-1-Cyclohexyl-3-[4-(2-methoxy-phenyl)cyclopentyl] piperazin;
*trans*-1-Cyclohexyl-4-[5-(2-methoxy-phenyl) 6-piperidyl] piperazin;
*trans*-1-Cyclohexyl-4-[4-(5-methoxy-naphtyl)cyclohexyl] piperazin,
oder den entsprechenden cis-Isomeren.

7. Ligand nach einem der Ansprüche 4 bis 6 zur Verwendung als Medikament.

8. Ligand nach Anspruch 7 in der Behandlung von Neoplasien, die durch die Überexpression der Histone H3, H2B, H2A.5, H1, H2.1 und des menschlichen 40S ribosomalen Proteins S3 gekennzeichnet sind.

9. Pharmazeutische Zusammensetzung, umfassend ein oder mehrere Liganden nach einem der Ansprüche 4 bis 6, einen pharmazeutisch verträglichen Hilfsstoff und gegebenenfalls übliche Zusatzstoffe.

10. Pharmazeutische Zusammensetzung nach Anspruch 9 in einer Rezeptur, die geeignet ist, Tumorzellen mittels Blockierung des Zyklus in der G0/G1-Phase und anschließende Apoptose zu behandeln.

11. Diagnostische Sonde umfassend den Komplex des Liganden nach einem der Ansprüche 4 bis 6 und ein Markermolekül, das ein erkennbares Signal emittieren kann.

12. Diagnostische Sonde nach Anspruch 11, wobei der Marker ein Fluoreszenzmolekül, ein Enzym, ein radioaktives Isotop ist.

13. Diagnostisches Verfahren zum Nachweis der Anwesenheit von soliden Tumoren oder zur Beobachtung ihrer Entwicklung, umfassend einen Schritt der *in vitro-*Bestimmung in einer Gewebebiopsie, des Expressionslevels eines oder mehrerer menschlicher Histone in einer teilweise deacetylierten Form, ausgewählt aus den Histonen H3, H2B, H2A.5, H1, H2.1 und des menschlichen 40S ribosomalen Proteins S3, wobei die Bestimmung der Expressionslevel unter Verwendung der Sonde nach Anspruch 12 durchgeführt wird.

14. Verfahren nach Anspruch 13, wobei die Gewebeprobe auf einem festen Träger immobilisiert ist und die Sonde eine Fluoreszenzsonde ist.

15. Verfahren nach einem der Ansprüche 13 oder 14, wobei der Tumor ein Neuroblastom, Urothelcarcinom, Brustcarcinom oder Gliom ist.

## Revendications

1. Procédé pour préparer des ligands spécifiques d'un récepteur de type sigma-2, comprenant les étapes consistant à
- mettre des composés ligands candidats en contact avec une ou plusieurs histones humaines sous une forme partiellement désacétylée, choisies parmi les histones H3, H2B, H2A.5, H1, H2.1 et/ou la protéine ribosomale 40S humaine S3 ;
- sélectionner le composé capable de générer un complexe de récepteur/ligand ;
- isoler le ligand du complexe, et éventuellement,
- purifier et identifier le ligand,
- vérifier l'activité apoptotique et cytotoxique intrinsèque du ligand sur des cellules de gliome ou de neuroblastome.

2. Procédé selon la revendication 1, dans lequel, en tant que récepteur, sont utilisées une ou plusieurs histones et/ou la protéine ribosomale 40S humaine S3, toutes sous une forme choisie parmi : la forme pure, la fraction de membrane cellulaire, la fraction de protéine nucléaire, et les cellules intactes naturelles ou génétiquement modifiées.

3. Procédé selon la revendication 2, dans lequel le récepteur sous la forme d'un mélange de protéines pures ou d'une fraction cellulaire ou de cellules intactes est en phase liquide ou est immobilisé sur une phase stationnaire.

4. Ligand spécifique du récepteur sigma-2, pouvant être obtenu par le procédé selon l'une quelconque des revendications 1 à 4, et ayant la formule générale 1 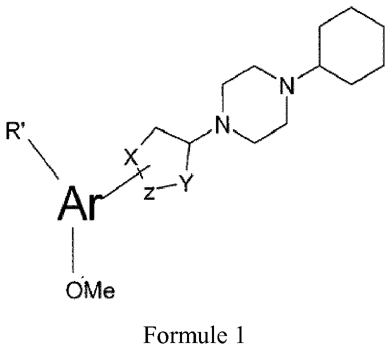 dans laquelle :
Ar est un groupe monocyclique à 5 ou 6 chaînons méthoxyaryle ou méthoxyhétéroaryle contenant un ou plusieurs hétéroatomes choisis parmi N, O, S, ou un groupe méthoxyaryle polycyclique condensé, éventuellement partiellement hydrogéné,
R' est choisi parmi H, OH, alcoxy en C₁ à C₅ linéaires ou ramifiés, alkyle en C₁ à C₅ linéaires ou ramifiés, halogénoalkyle en C₁ à C₅ linéaires ou ramifiés, les halogènes, NO₂, CH₂OH, NHCH₃, N(CH₃)₂, CONH₂, NHSO₂CH₃ et COCH₃ ;
Z est choisi parmi -CH₂-, -CH<, -C₂H₄-, et -C₂H₃< ;
X et Y sont choisis parmi N, O et S,
et où chaque motif cyclique peut être sous forme cis ainsi que sous forme trans.

5. Ligand selon la revendication 4, dans lequel Ar est un résidu 2-méthoxyphényle, 2-méthoxy-6-aminophényle, 2-méthoxy-5-aminophényle, 2-méthoxy-5-aminophényle, 2-méthoxy-6-nitrophényle.

6. Ligand selon la revendication 4, choisi parmi :
la *trans*-1-cyclohexyl-4-[4-(2-méthoxyphényl)cyclohexyl]pipérazine ;
la *trans*-1-cyclohexyl-4-[4-(2-méthoxy-6-éthylphényl)cyclohexyl]pipérazine ;
la *trans*-1-cyclohexyl-4-[4-(2-méthoxy-6-aminophényl)cyclohexyl]pipérazine ;
la *trans*-1-cyclohexyl-4-[4-(2-méthoxy-5-méthylaminophényl)cyclohexyl]pipérazine ;
la *trans*-1-cyclohexyl-3-[4-(2-méthoxyphényl)cyclopentyl]pipérazine ;
la *trans*-1-cyclohexyl-4-[5-(2-méthoxyphényl)-6-pipéridyl]pipérazine ;
la *trans*-1-cyclohexyl-4-[4-(5-méthoxynaphtyl)cyclohexyl]pipérazine,
et les isomères *cis* correspondants.

7. Ligand selon l'une quelconque des revendications 4 à 6, pour utilisation en tant que médicament.

8. Ligand selon la revendication 7, pour le traitement de néoplasies **caractérisées par** une surexpression des histones H3, H2B, H2A.5, H1, H2.1 et de la protéine ribosomale 40S humaine S3.

9. Composition pharmaceutique comprenant un ou plusieurs ligands selon les revendications 4 à 6, un excipient pharmaceutiquement acceptable et, éventuellement, des additifs habituels.

10. Composition pharmaceutique selon la revendication 9, dans une formulation convenant pour le traitement de cellules tumorales par blocage du cycle dans la phase G0/G1 et apoptose subséquente.

11. Sonde de diagnostic comprenant le complexe du ligand selon l'une quelconque des revendications 4 à 6 et une molécule marqueur capable d'émettre un signal reconnaissable.

12. Sonde de diagnostic selon la revendication 11, dans laquelle le marqueur est une molécule fluorescente, une enzyme, un isotope radioactif.

13. Méthode de diagnostic pour la détection de la présence de tumeurs solides ou pour la surveillance de leur développement, comprenant une étape de détermination *in vitro*, sur biopsie tissulaire, du niveau d'expression d'une ou plusieurs histones humaines sous une forme partiellement désacétylée, choisies parmi les histones H3, H2B, H2A.5, H1, H2.1, et de la protéine ribosomale 40S humaine S3, dans laquelle la détermination des niveaux d'expression est effectuée par utilisation de la sonde selon la revendication 12.

14. Méthode selon la revendication 13, dans laquelle l'échantillon tissulaire est immobilisé sur un support solide et la sonde est une sonde fluorescente.

15. Méthode selon l'une quelconque des revendications 13 et 14, dans laquelle la tumeur est un neuroblastome, un carcinome urothélial, un carcinome mammaire, un gliome.
